# EUROPEAN PATENT APPLICATION

(11) **EP 3 582 230 A1**
(43) Date of publication of application: **18.12.2019**
(21) Application number: 18177213.8
(22) Date of filing: 12.06.2018
(51) Int. Cl.: G16H 50/20

(54) **EDGE-COMPUTING UNIT, MEDICAL ANALYTICS CLOUD-COMPUTING NETWORK, AND METHOD FOR PERFORMING STATISTICAL ANALYSIS**

(71) Applicant: Bittium Biosignals Oy, 70800 Kuopio (FI)
(72) Inventor: Sarén, Matti, 87250 Kajaani (FI); Remes, Arto, 70800 Kuopio (FI)
(74) Representative: LEITZINGER OY

(57) **Abstract**

The present disclosure describes for a medical analytics cloud-computing network, an edge-computing unit for the cloud-computing network, and a method for performing statistical analysis in cloud-computing network. The cloud computing network comprises at least one analytics engine within the cloud-computing network, and at least one edge-computing unit at the edge of the cloud computing network. The edge-computing unit is configured to receive a data processing task originating from the at least one analytics engine, perform the data processing task to extract the at least one feature, and return the extracted feature to be received by the analytics engine.

## Description

### FIELD

The invention relates to analysis of medical data, and in particular, to systems for analysing medical data.

### BACKGROUND INFORMATION

Advances in computer calculating power have enabled processing of large amount of data in short amount of time, or even in real time. With large amounts of data, new correlations between measurable parameters, features and/or sequences in biosignal data and physical conditions (e.g. a disease) can be found. Biosignal data may represent samples of measurements of biosignals of persons, for example. Electroencephalography (EEG), electromyography (EMG) and electrocardiography (ECG) are some examples of electrically measurable biosignals. The correlations may relate to generic population or to specific populations representing a group of a specific age, weight, gender, and/or social background, for example. These correlations may then be used in determining and/or confirming the presence of a condition in a patient. As large amounts of data may have to be analysed in order to be able to find new correlations, patient data of one hospital, or even all hospitals of a region / nation may not be enough for the required statistical analysis. It may therefore be useful to be able to combine different sources of patient data. By combining different data sources, a far more extensive view on the patient data can be formed.

However, data privacy issues have to be taken into account when accessing such data. For example, legislation of countries/regions may prohibit export and use of personal data of their citizens abroad. Personal data may be defined as any information relating to an identified or identifiable natural person, where an identifiable person is one who can be identified directly or indirectly, in particular by reference to an identification number or to one or more factors specific to his physical, physiological, mental, economic, cultural or social identity. Data may be considered "personal data" when someone is able to link the information to a person, even if the person holding the data cannot make this link.

As access to personal data as defined above may be severely restricted, it may be difficult to utilize the large amounts of data stored in different countries/regions.

### BRIEF DISCLOSURE

An object of the present disclosure is to provide a method for analysing medical data and a system for implementing the method so as to alleviate the above disadvantages. The object of the disclosure is achieved by an edge-computing unit, an analytics engine and a cloud computing network which are characterized by what is stated in the independent claims. The preferred embodiments of the disclosure are disclosed in the dependent claims.

The present disclosure describes a medical analysis method that utilizes edge-computing. The method may implement an edge-computing system distributes data processing tasks to edge-computing units at the edge of a computing cloud. The data processing tasks may relate to local data accessible by the edge-computing units. For example, the data processing task may relate to a statistical analysis of biosignals data stored locally by a hospital. Once the edge-computing units have performed the tasks assigned to them, the results of the tasks are gathered and used to form an extensive view based on the whole data.

The gathered results may represent features extracted from the local data but the data processing tasks can be formulated such that the features extracted by performing the tasks do not represent identifiable data that can be retroactively linked back to an individual person. Thus, no personal data is exported and violations to data privacy legislation can be avoided.

### BRIEF DESCRIPTION OF THE DRAWINGS

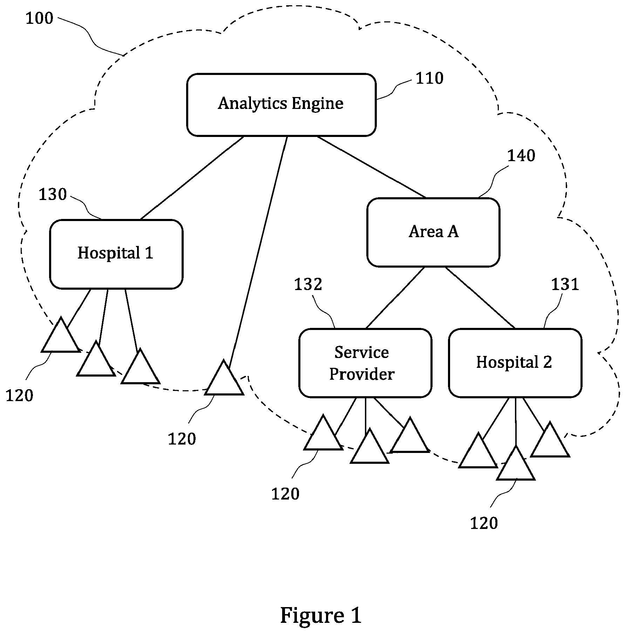

In the following the invention will be described in greater detail by means of preferred embodiments with reference to the attached drawings, in which
Figure 1 shows an exemplary embodiment of a medical analytics cloud-computing network according to the present disclosure.

### DETAILED DISCLOSURE

The present disclosure describes a method for performing statistical analysis on medical data in a cloud-computing network. The medical data may be in the form of biosignal data representing stored samples of a measured biosignal, for example. The method comprises sending a data processing task to be received by at least one edge-computing unit at the edge of the cloud computing network, receiving at least one result originating from the at least one edge-computing unit performing the data processing task, and performing the statistical analysis on the basis of the at least one result.

The data processing task may be intended for extracting a feature of local medical data, e.g. a feature of medical data only accessible locally by edge-computing units affiliated with a medical entity, such as a hospital, storing the data. For example, a data processing task in a method according to the present disclosure may be a distributed computational task of a statistical analysis of medical data. Said distributed computational task may be intended for extracting at least one feature of the biosignal data, for example. A feature may be a representation of characteristics of a biosignal calculated on the basis of the biosignal data, for example. Heartbeat inverval, heartbeat variability, and characteristics calculated from a P wave (that shows atrial activity during a single heart beat) can be such features of a ECG signal, for example. The distributed computational task and its result, i.e. the at least one extracted feature, may be formulated such that the biosignal data or its origin (e.g. identity of a patient from whom the biosignal data was measured) cannot be reconstructed based on the at least one feature. A data processing task received by the edge-computing unit according to the present disclosure may be in the form of instructions that describe operations that the edge-computing unit should perform on the local medical data. For example, the instruction may describe a statistical operation to be performed on biosignal data that is locally accessible by the edge-computing unit.

For performing a data processing task according to the present disclosure, the present disclosure also describes a complementing method. Said method is intended for performing a distributed computational task of a statistical analysis on medical data in cloud-computing network. The method comprises receiving a data processing task according to the present disclosure on at least one edge-computing unit at the edge of the cloud computing network, performing the data processing task on the at least one edge-computing unit in order to extract the at least one feature from the medical data, and returning the extracted feature to be received an analytics engine configured to perform the statistical analysis on the basis of the at least one extracted feature.

In order to implement the above-discussed methods for statistical analysis according to the present disclosure, a medical analytics system may be formed. The medical analysis system may be a medical analysis cloud computing network comprising one or more edge-computing units at the edge of the cloud computing network, and one or more analytics engines within the cloud-computing network, for example. Preferably, there are a plurality of the edge-computing units, such as ten or more, a hundred or more, or a thousand or more in the cloud computing network. Figure 1 shows an exemplary embodiment of a medical analytics cloud-computing network according to the present disclosure. In Figure 1, a cloud computing network 100 and an analytics engine 110 within the cloud-computing network 100 are shown. Figure 1 also shows a plurality of edge-computing units 120 (depicted as triangles) at the edge of the cloud computing network 100.

In the context of the present disclosure, an edge-computing unit may be a unit comprising means configured to receive a data processing task according to the present disclosure, perform the data processing task to extract at least one feature of locally accessible medical data, and return the extracted feature to be received by the analytics engine in order for the analytics engine to perform the statistical analysis on the basis of the extracted at least one feature. For example, Figure 1 shows three unaffiliated entities: Hospital 1 with reference number 130; Hospital 2 with reference number 131; and Service Provider with reference number 132. Each of these entities store may store personal data that is not accessible by other entities. Each entity 130 - 132 is connected to a plurality of edge-computing device 120 according to the present disclosure in Figure 1. Some (or all) of the edge-computing units 120 may be dedicated computer servers or a virtual machines configured to act as the edge-computing unit. The server or virtual machine may be configured to read data from medical devices and perform the data processing task on the basis of this data.

The edge-computing units 120 may also be a smart device, such a smart sensor attached to a patient or a smart medical device operated by medical personnel. The smart device may comprise a computing unit configured to receive and perform distributed computational tasks originating from an analytics engine. The computing unit may be a computing device, such as a processor, a DSP, an FPGA, or an ASIC, coupled with a computer memory, for example. The smart device may further comprise communications unit, which preferably is a wireless communications unit, such as a WLAN or Bluetooth module. The distributed computational tasks and the results of such tasks may be communicated via the communications unit. In Figure 1, some (or all) of the edge-computing devices may be such smart devices.

The above-discussed data processing task may originate from an analytics engine. In the context of the present disclosure, an analytics engine according to the present disclosure may be an algorithm running in a cloud computing network, for example. The algorithm may run on one or more servers or virtual machines, for example.

The analytics engine may be configured to act as a tool for diagnostics and for managing data logistics. The analytics engine may also utilize machine learning. The analytics engine may distribute machine learning tasks to edge-computing units according to the present disclosure. Based on the results provided by the edge-computing units, a solution to the machine learning task may be resolved.

In Figure 1, the analytics engine 110 may be configured to perform the statistical analysis on the basis of at least one extracted feature originating from the at least one edge-computing unit 120. Although Figure 1 shows one analytics engine 110, there may also be more than one analytics engines running in parallel in the cloud computing network 100. The parallel analytics engines may each communicate with a subset of edge-computing devices at the edge of the cloud computing network. The subset may comprise one to all edge-computing units accessible via the cloud computing network. In some embodiments, none of the edge-computing units in the subsets of different analytics engines are the same. In other embodiments, the subsets may have some overlapping edge-computing units, or the subsets may even be identical.

The analytics engine and the one or more edge-computing units may be connected to each other via a cascade computing system that forms a hierarchical structure starting from the analytics engine and branching out towards the one or more edge-computing devices at the edge of the cloud, thereby effectively forming a tree-like hierarchy of data processing nodes where the analytics engine is a root node and the edge-computing units are leave nodes. As shown in Figure 1, the analytics engine 120 may be directly connected to the edge-computing units in the tree-like structure. There may also be data processing nodes between the analytics engine and the edge-computing units in the cascade computing system, as also shown in Figure 1. Such an intermediate data processing node may be a dedicated server or a virtual machine, for example. Thus, there may be zero, one, or more intermediate data processing nodes between the analytics engine and an edge-computing unit in the tree-like hierarchy of a cascade computing system according to the present disclosure.

The analytics engine may distribute a data processing task to its descendant computing node or nodes in the tree-like hierarchy. From these nodes, the data processing task may be distributed to further descendant nodes and so on until the task is received by an edge-computing unit configured to receive such a task. The results of the data processing tasks performed on the edge-computing devices may be routed back to the analytics engine in the same cascaded manner.

As discussed above, the data processing task may be in the form of instructions describing the operations to be performed. In some embodiments, the instructions may be in the form of a software code that is executable on the edge-computing unit. In another embodiment, the instructions may define higher level mathematical operations, such as statistical functions, to be performed on the data, and the edge-computing unit may be configured to interpret these instructions into software code that is then executed on the edge-computing unit.

Analytics engines according to the present disclosure and data processing tasks generated by the analytics engines may be intended for achieving various goals. For example, a medical analytics system according to the present disclosure may drive for finding new correlations / patterns between biosignal data and specific populations. Machine learning may be used for modelling relations between features and patterns of biosignals and symptoms of various conditions of human body, for example. As medical data can be accessed without compromising data privacy of patients, medical data of unaffiliated medical facilities, even across national borders can be used in the analysis. In the following, some of exemplary uses of the medical analysis system according to the present disclosure are described.

A medical analysis system as discussed above may be configured to estimate level of consciousness (LOC) of a person, for example. As the system may have access to EEG data of thousands of patients of specific groups instead of tens of patients, a more accurate model of the level of consciousness may be developed. Similarly, the large amount of EEG data may be used for improving the model for determining brain symmetricity index, for example. In a similar manner, improved models for detecting different heart arrhythmia in specific groups of patients may be developed with the medical analytics system as discussed above.

The medical analytics system as discussed above may also be used to implement a monitoring system for monitoring usage of medical devices. The monitoring system may comprise at least one edge-computing unit according to the present disclosure. In the monitoring system, the at least one edge-computing unit is configured to receive a data processing task giving instructions on how to determine a value representing the usage of the edge-computing unit or at least one other unit accessible by the edge-computing unit. For example, if the edge-computing unit is a smart measurement sensor or a smart medical device operated by medical personnel, the data processing task may instruct the smart medical device to calculate its usage, e.g. in the form of hours or minutes. The smart device may also be instructed to determine how it has been used, if the smart device has different operating modes, e.g. in the form of different measurements. Alternatively, the edge-computing unit may be connected to a sensor or a medical device without edge-computing capabilities and gather the required usage information from them.

The usage-related data processing task may originate from an analytics engine, and once the edge computing unit has performed the task, it may send the results to be received by the analytics engine. The analytics engine may perform the statistical analysis on the basis of the results. For example, the statistical analysis may be used for determining which the most used operating modes are, and this information may be used for further improving the smart sensors and smart medical devices. The results on usage may also be used for billing. The edge-computing devices or devices accessible by the edge-computing devices may be available for a service provider on a contract of lease, and the monthly costs may be determined on the basis of the usage.

The medical analytics system as discussed above may also be used to implement an update delivery system. The update delivery system may comprise at least one edge-computing unit according to the present disclosure. In the update delivery system, the at least one edge-computing unit may be configured to receive a data processing task that give instructions on how to update a software or firmware of the edge-computing unit or a device connected to the edge-computing unit. In this manner, an update may be delivered to all desired units / devices in a centralized manner.

It is obvious to a person skilled in the art that the electrode patch and the detection method/system can be implemented in various ways. The invention and its embodiments are not limited to the examples described above but may vary within the scope of the claims.

## Claims

1. An edge-computing unit for a medical analytics cloud-computing network, the cloud computing network comprising
at least one analytics engine within the cloud-computing network, and
the edge-computing unit at the edge of the cloud computing network, wherein the edge-computing unit is configured to
- receive a data processing task originating from the at least one analytics engine, wherein the data processing task is a distributed computational task of statistical analysis of biosignal data to extract at least one feature of the biosignal data, wherein the at least one feature is such that the biosignal data or its origin cannot be reconstructed based on the at least one feature,
- perform the data processing task to extract the at least one feature, and
- return the extracted feature to be received by the analytics engine in order for the analytics engine to perform the statistical analysis on the basis of the at least one extracted feature.

2. A medical analytics cloud-computing network comprising
an least one edge-computing unit according to claim 1, and
an analytics engine within the cloud-computing network, the analytics engine being configured to perform the statistical analysis on the basis of the extracted at least one feature.

3. An medical analytics cloud-computing network comprising
an analytics engine within the cloud-computing network, and at least one edge-computing unit at the edge of the cloud computing network, wherein each of the at least one edge-computing unit is configured to
- receive a data processing task originating from the analytics engine, wherein the data processing task is a distributed computational task of a statistical analysis of a biosignal data to extract a at least one feature of the biosignal data, wherein the at least one feature is such that the biosignal data or its origin cannot be reconstructed based on the at least one feature,
- perform the data processing task to extract the at least one feature,
and
- return the at least one extracted feature to be received by the analytics engine,
and wherein the analytics engine is configured to
- perform the statistical analysis on the basis of the at least one extracted feature.

4. A method for performing statistical analysis on biosignal data in cloud-computing network, the method comprising
- sending a data processing task to be received by at least one edge-computing unit at the edge of the cloud computing network, wherein the data processing task is a distributed computational task of a statistical analysis of a biosignal data to extract a at least one feature of the biosignal data, wherein the at least one feature is such that the biosignal data or its origin cannot be reconstructed based on the at least one feature,
- receiving at least one extracted feature originating from the at least one edge-computing unit performing the data processing task, and
- performing the statistical analysis on the basis of the at least one extracted feature.

5. A method for performing a distributed computational task of a statistical analysis on biosignal in cloud-computing network, the method comprising
- receiving a data processing task to on at least one edge-computing unit at the edge of the cloud computing network, wherein the computational task is a distributed computational task of a statistical analysis of a biosignal data to extract at least one feature of the biosignal data, wherein the at least one feature is such that the biosignal data or its origin cannot be reconstructed based on the feature,
- performing the data processing task on the at least one edge-computing unit in order to extract the at least one feature, and
- returning the extracted feature to be received an analytics engine configured to perform the statistical analysis on the basis of the at least one extracted feature.
